# EUROPEAN PATENT APPLICATION

(11) **EP 4 721 648 A1**
(43) Date of publication of application: **08.04.2026**
(21) Application number: 25206506.5
(22) Date of filing: 02.10.2025
(51) Int. Cl.: A61B 5/00, A61B 5/257

(54) **SKIN ADHESIVE SYSTEM**

(30) Priority: 02.10.2024 US 202463702366 P; 09.12.2024 US 202463729734 P
(71) Applicant: Bardy Diagnostics, Inc., Bellevue, WA 98005 (US)
(72) Inventor: FLOYD, Jared, Bellevue, 98005 (US); REDDY, Daniel, Bellevue, 98005 (US); ELLIOT, Joel, I, Bellevue, 98005 (US); PETERSON, Mercer, Bellevue, 98005 (US); CRAN, Brian, Bellevue, 98005 (US)
(74) Representative: Reddie & Grose LLP

(57) **Abstract**

An adhesive system for a wearable medical device is provided. The adhesive system for a includes an electrode patch with one or more electrodes for sensing a signal indicative of a physiological characteristic of a patient. The adhesive system further includes a removable section disposed at least partially around a perimeter of the electrode patch that is configured to be removed from the adhesive system.

## Description

### PRIORITY CLAIM AND CROSS-REFERENCE TO RELATED APPLICATIONS

This application claims priority to, and the benefit of, U.S. Provisional Patent Application No. 63/702,366, filed October 2, 2024, titled "SKIN ADHESIVE SYSTEM," and U.S. Provisional Patent Application No. 63/729,734, filed December 9, 2024, titled "PERFORATED SKIN ADHESIVE SYSTEM," the entire contents of each of which are incorporated by reference herein in their entirety and relied upon.

### FIELD

This application relates to a skin adhesive system for wearable medical devices.

### BACKGROUND

Wearable medical devices are used in a variety of applications. Such non-limiting uses of wearable medical devices include patient monitoring, delivery of patient therapeutics, and medical diagnosis. In one example, a patient wears an electrocardiogram ("ECG") monitor to measure and record electrical signals emitted from the heart. Conventionally, the ECG monitor uses a standardized set format lead configuration to record electrical signals. Electrodes often include an adhesive on one of their sides, which permits the electrodes to adhere to the skin of the patient at a desired position on the body. While an ECG monitor is described as one example, many other wearable medical devices rely on adhesive systems in various applications.

Once the wearable medical device is properly attached to the patient, the device may collect data via patient monitoring. This collected data can then be used to determine a patient's physiology through various medical diagnostic procedures. For example, data collected by an ECG monitor can be used to diagnose arrhythmias or other related cardiac diseases. Generally, the adhesive system that couples the medical device to the patient can affect the amount, such as the duration/length of data, and quality, due to proper/improper adhesion, of data collected, which, in turn, may affect diagnostic efficacy. Accordingly, diagnostic efficacy can be improved, when appropriate, through effective long-term monitoring.

A need remains for adhesive systems for wearable medical devices that appropriately couple medical devices to the patient for a desired period.

### SUMMARY

An adhesive system for coupling a wearable medical device to a patient is provided. The adhesive system includes a tear-away portion, which may be selectively removed from the adhesive system. In an example, the edge of the adhesive system may gradually lift. To prevent further failure, the tear-away portion may be removed, providing a new edge that is firmly adhered to the patient's skin. In turn, the adhesive system of the present disclosure prevents the lifted edge from propagating, worsening, or causing the wearable medical device to fall-off before the prescribed removal date. Therefore, the adhesive system of the present disclosure may increase the patient monitoring period, which may increase diagnostic efficacy. With such improved diagnostic efficacy, the quality of patient care may increase.

Additionally or alternatively, the adhesive system of the present disclosure may include a plurality of openings. Such openings create an unimpeded conduit for moisture egress, thereby reducing moisture buildup between the adhesive system and the skin. By reducing moisture buildup, the adhesive system may increase the breathability of the system and prevent degradation of the adhesive bond from moisture pooling. With an increase of breathability, the adhesive system provides various advantages. For example, an adhesive system with adequate breathability may provide additional patient comfort. Rather than retaining moisture, perspiration is released, lowering the temperature of the skin and also lowering the chances of skin irritation. In turn, patient compliance may increase, allowing the wearable medical device to collect data over a longer period of time.

Selective removal of various sections of an adhesive system, such as a tear-away portion and/or an opening may further provide for customization of the adhesive system on a patient-by-patient basis. Customization would only further lead to increased comfort, and thus an increase in the overall patient monitoring period.

In light of the disclosure set forth herein, and without limiting the disclosure in any way, in a first aspect of the present disclosure, which may be combined with any other aspect, or portion thereof, an adhesive system for a wearable medical device includes an electrode patch and a removable section. The electrode patch includes one or more electrodes for sensing a signal indicative of a physiological characteristic of a patient. The removable section is disposed at least partially around a perimeter of the electrode patch. The removable section comprises an adhesive. The removable section is configured to be removed from the adhesive system.

In a second aspect of the present disclosure, which may be combined with any other aspect, or portion thereof, the removable section comprises a first removable section and a second removable section, wherein the first removable section is disposed at least partially around a perimeter of the electrode patch and the second removable section is disposed at least partially around a perimeter of the first removable section.

In a third aspect of the present disclosure, which may be combined with any other aspect, or portion thereof, the electrode patch includes a distal end and a proximal end. The removable section includes a distal removable section disposed around the distal end of the electrode patch, and a proximal removable section disposed around the proximal end of the electrode patch.

In a fourth aspect of the present disclosure, which may be combined with any other aspect, or portion thereof, the distal end of the electrode patch comprises a first electrode and the proximal end of the electrode patch comprises a second electrode.

In a fifth aspect of the present disclosure, which may be combined with any other aspect, or portion thereof, the distal removable section and the proximal removable section are independently removable.

In a sixth aspect of the present disclosure, which may be combined with any other aspect, or portion thereof, the removable section includes a contact portion and an overlap portion. The contact portion has a contact inner surface, the contact inner surface is configured to adhere to skin of a patient. The overlap portion has an overlap inner surface, the overlap inner surface is configured to adhere to the electrode patch.

In a seventh aspect of the present disclosure, which may be combined with any other aspect, or portion thereof, the contact inner surface has a stronger adhesive than the overlap inner surface, such that the overlap inner surface is permanently adhered to the electrode patch.

In an eighth aspect of the present disclosure, which may be combined with any other aspect, or portion thereof, the removable section comprises one or more tabs, and the removable section has a thickest width at the one or more tabs.

In a ninth aspect of the present disclosure, which may be combined with any other aspect, or portion thereof, the one or more tabs comprises a first tab and a second tab.

In a tenth aspect of the present disclosure, which may be combined with any other aspect, or portion thereof, the electrode patch and the removable section are co-planar.

In an eleventh aspect of the present disclosure, which may be combined with any other aspect, or portion thereof, a plurality of perforations are disposed on an interface of the electrode patch and the removable section.

In a twelfth aspect of the present disclosure, which may be combined with any other aspect, or portion thereof, the removable section overlaps a portion of the perimeter of the electrode patch.

In a thirteenth aspect of the present disclosure, which may be combined with any other aspect, or portion thereof, the adhesive system further includes an additional removable section surrounded by a plurality of perforations, wherein the additional removable section creates an opening when removed from the adhesive system.

In a fourteenth aspect of the present disclosure, which may be combined with any other aspect, or portion thereof, a wearable medical device includes an electrode patch, a monitor recorder, and an adhesive system. The electrode patch includes one or more electrodes for sensing a signal indicative of a physiological characteristic of a patient. The monitor recorder has a microcontroller, the monitor recorder configured to receive the signal from the one or more electrodes. The adhesive system includes an adhesive configured to adhere to a skin of the patient. The adhesive system is disposed at least partially around a perimeter of the electrode patch. The adhesive system comprises a section that is configured to be removed from the adhesive system.

In a fifteenth aspect of the present disclosure, which may be combined with any other aspect, or portion thereof, the monitor recorder is removably coupled to the electrode patch.

In a sixteenth aspect of the present disclosure, which may be combined with any other aspect, or portion thereof, the section configured to be removed comprises a first removable section and a second removable section, and the first removable section is disposed at least partially around a perimeter of the electrode patch and the second removable section is disposed at least partially around a perimeter of the first removable section.

In a seventeenth aspect of the present disclosure, which may be combined with any other aspect, or portion thereof, the adhesive system comprises a plurality of perforations at an interface of the first removable section and the second removable section.

In an eighteenth aspect of the present disclosure, which may be combined with any other aspect, or portion thereof, the first removable section and the second removable section are co-planar.

In a nineteenth aspect of the present disclosure, which may be combined with any other aspect, or portion thereof, the adhesive system overlaps the electrode patch.

In a twentieth aspect of the present disclosure, which may be combined with any other aspect, or portion thereof, the adhesive system overlaps an entire perimeter of the electrode patch.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### BRIEF DESCRIPTION OF THE DRAWINGS

Figure 1 illustrates a perspective view of an extended wear electrocardiography monitor, according to various embodiments.
Figure 2 illustrates a front facing view of an extended wear electrocardiography monitor having an adhesive system, according to various embodiments.
Figure 3 illustrates a front facing view of an extended wear electrocardiography monitor having an adhesive system, according to various embodiments.
Figure 4 illustrates a perspective view of an extended wear electrocardiography monitor having an adhesive system, according to various embodiments.
Figures 5A to 5D illustrate an example process of a tear-away being removed from an adhesive system, according to various embodiments.
Figure 6 illustrates a cross-sectional view of an extended wear electrocardiography monitor having an adhesive system, according to various embodiments.
Figure 7 illustrates a detailed view of an adhesive system of the extended wear electrocardiography monitor of Figure 6, according to various embodiments.
Figure 8 illustrates a cross-sectional view of an extended wear electrocardiography monitor having an adhesive system, according to various embodiments.
Figure 9 illustrates a detailed view of an adhesive system of the extended wear electrocardiography monitor of Figure 8, according to various embodiments.
Figures 10A to 10B illustrates an extended wear electrocardiography monitor having a customizable adhesive system, according to various embodiments.
Figure 11 illustrates an extended wear electrocardiography monitor having nonessential material removed from a customizable adhesive system, according to various embodiments.
Figure 12 illustrates an extended wear electrocardiography monitor having nonessential material removed from a customizable adhesive system, according to various embodiments.
Figure 13 illustrates an extended wear electrocardiography monitor having a customizable adhesive system as applied to a patient's skin, according to various embodiments.
Figure 14 illustrates an extended wear electrocardiography monitor having a customizable adhesive system as applied to a patient's skin, according to various embodiments.
Figure 15 illustrates an extended wear electrocardiography monitor having applied to a patient's skin, according to various embodiments.
Figure 16 illustrates an extended wear electrocardiography monitor having a plurality of openings applied to a patient's skin, according to various embodiments.
Figure 17 illustrates a front-facing view of an extended wear electrocardiography monitor having a plurality of openings, according to various embodiments.

### DETAILED DESCRIPTION

As previously introduced, wearable medical devices may provide patients with various benefits, including continuous monitoring of physiological signals. Data collected through continuous monitoring can be used, for example, to detect sporadic events or facilitate diagnosis by a medical professional. Diagnostic efficacy can be affected by the quality and amount of data collected. For example, an increase in the amount of data collected allows a physician (or diagnostic software) to identify events of potential concern that may not otherwise be detected during a shorter monitoring period. In another example, a physician could more accurately determine the severity of a diagnosis by analyzing the number of occurrences over a longer period. Therefore, by increasing the amount of data collected through long-term monitoring, diagnostic efficacy can improve. With improved diagnostic efficacy, the quality of patient care will increase.

While this described monitoring is often essential to provide a high quality of patient care, continuous monitoring over an extended period is challenging. One challenge in continuous monitoring is appropriately coupling the wearable medical device to the patient. Typically, wearable medical devices include one or more electrodes. A patient may secure the one or more electrodes to their body using an adhesive system provided with the electrode. In some instances, the adhesive coupling the wearable medical device to the patient can prematurely fail, affecting the electrode placement. If the electrodes do not maintain contact with the patient (e.g., with the patient's skin), the signal obtained from the electrodes may be inaccurate or may not be recorded at all. Thus, the patient cannot benefit from the application of the wearable medical device.

In some examples, the adhesive systems may gradually fail over time. Often, this gradual failure begins at the edges of the adhesive system before propagating to other areas, leading to complete failure of the adhesive system. Alternatively, this gradual failure could begin at uneven surfaces of the skin that create gaps between the adhesive and the skin. In one example, foreign substances may build up around the edges of the adhesive system. Such build up, in combination with repeated friction caused by the movement of clothing or the patient's skin acting as the substrate, causes the edges of the adhesive system to gradually lift. Once an edge has begun to lift, the edge of the adhesive system becomes more vulnerable to catching on clothing or other parts of the patient's body during daily activities. Catching will cause the cycle to repeat, or worsen, as the edge of the adhesive system continues to lift until eventually, the lifted edge causes the adhesive system to completely fail before a prescribed removal date.

Additionally, breathability is one feature that may affect whether the adhesive system fails to appropriately couple the wearable medical device to the patient. For example, without adequate breathability, moisture from patient perspiration can collect between the wearable medical device and the skin at the adhesive surface. Over time, the moisture may pool, causing the adhesive surface to separate from the skin. When the adhesive surface separates from the skin, the adhesive bond to the skin will degrade, increasing the likelihood of a premature failure. Lack of appropriate breathability may also cause undesirably high temperature at the adhesive surface. Such high temperature may enhance patient perspiration, further degrading the adhesive bond between the wearable medical device and the patient.

The present disclosure provides an adhesive system designed to prevent or limit gradual failure of the adhesive system.

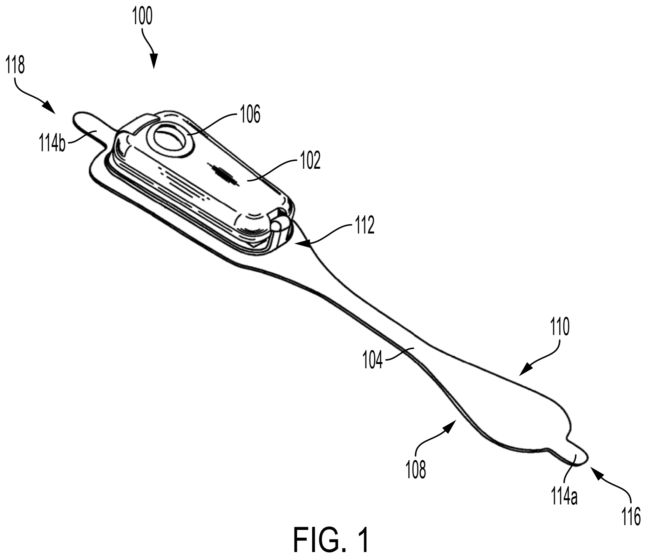

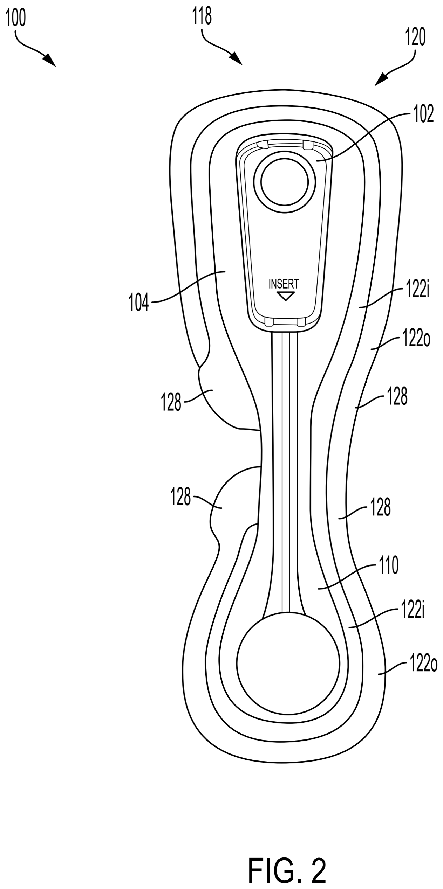

Figure 1 illustrates a perspective view of an extended wear electrocardiography monitor, according to various embodiments. The extended wear electrocardiography monitor ("wearable monitor") 100 of Figure 1 is an example of a wearable medical device of the present disclosure, which uses an adhesive system. Additional details of the adhesive system are provided in reference to Figures 2 to 16. It should be appreciated that the wearable monitor 100 provides one example of a wearable medical device in which the adhesive system of the present disclose may be applied. The adhesive system may be applied to any number of other wearable medical devices that rely on adhesive systems to couple the wearable medical device to the patient.

The wearable monitor 100 includes a monitor recorder 102, which is removably coupled to an electrode patch 104 during use. The monitor recorder 102 contains electronic circuitry for recording and storing the patient's physiological signals as sensed by electrodes (not shown) disposed on the electrode patch 104. In an example embodiment, the electronic circuitry of the monitor recorder 102 includes a microcontroller, a memory, an electrocardiogram signal processor, and an analog-to-digital converter. The monitor recorder 102 may also include an external patient control. For example, the monitor recorder 102 of Figure 1 includes a button 106. In use, the patient may press the button 106 to mark a potential cardiac event. Additional details of the wearable monitor 100 are further described in commonly assigned U.S. Patent No. 11,701,044, issued July 18, 2023, the disclosure of which is incorporated by reference in its entirety.

The electrode patch 104 includes an adhesive for adhering the wearable monitor 100 to the patient's skin. Additionally, the electrode patch 104 includes one or more electrodes for sensing physiological signals of the patient. For example, the electrode patch 104 in the depicted embodiment includes a first electrode on a distal end 118 of the electrode patch 104 and a second electrode on a proximal end 116 of the electrode patch 104. When the wearable monitor 100 is adhered to the patient, the electrodes directly or indirectly contact the patient's skin for sensing.

In reference to Figure 1, the electrode patch 104 includes an inner side 108 and an outer side 110. When coupled to the patient, the inner side 108 includes the adhesive, which contacts and adheres to the patient's skin. The inner side 108 further includes the electrodes, configured to contact the patient's skin surface. The outer side 110 faces outward, away from the from the patient's skin and includes an attachment mechanism 112 for electrically coupling the monitor recorder 102 to the electrode patch 104 during use. Based on such coupling, the electrode patch 104 may collect physiological signals (e.g., ECG signals) from the electrodes, which are transmitted to the monitor recorder 102. However, without proper attachment of the electrode patch 104 to the patient, the electrode patch 104 cannot accurately collect physiological signals and thus the monitor recorder 102 will not receive relevant physiological signals for subsequent processing, transmission, or diagnostic procedures. In turn, the patient cannot benefit from the application of the wearable monitor 100.

The electrode patch 104 may further include placement tabs 114a, 114b. In reference to Figure 1, the electrode patch 104 includes a first placement tab 114a disposed on a proximal end 116 of the electrode patch 104 and a second placement tab 114b on a distal end 118 of the electrode patch 104. As previously introduced, the inner side 108 of the electrode patch 104 contacts and adheres to the patient's skin, such as through the use of an adhesive. To provide ease of placement, the placement tabs 114 may lack adhesive on the inner surface. In an illustrative example, the placement tabs 114 provide a non-adhesive component that the patient can grip while placing the inner side 108 of the electrode patch 104 onto their skin, ensuring proper orientation. In further embodiments, the location, size, and shape of the placement tabs 114 may be adjusted. Additionally, perforations may be configured such that the patient can remove the placement tabs 114 from the electrode patch 104 once properly adhered to the skin. While the present disclosure refers to the application of the wearable monitor 100 from the perspective of the patient, it should be appreciated that the wearable monitor 100 may be applied to the patient by any number of people, including a physician or caretaker.

Figure 2 illustrates a front facing view of an extended wear electrocardiography monitor having an improved adhesive system, according to various embodiments. The wearable monitor 100 includes the monitor recorder 102 and the electrode patch 104. The front facing view depicts the outer side 110 of the electrode patch 104 as described in reference to Figure 1. In reference to Figure 2, the wearable monitor 100 further includes an adhesive system 120, which is configured to prevent or limit gradual failure of the wearable monitor 100 through selective removal of a section of the adhesive system 120. In some embodiments, the adhesive system 120 includes the electrode patch 104. In some embodiments, the adhesive system 120 and the electrode patch 104 are separate components. Removing a section 122 may provide an edge (e.g., a new edge) that is firmly adhered to the patient's skin, leading to an increased monitoring period. With an increased monitoring period, the wearable monitor 100 may collect additional data for improved diagnostic efficacy.

The adhesive system 120 includes at least one section 122 configured to be removed or torn away from the adhesive system 120. In an example embodiment, as illustrated in Figure 2, the adhesive system 120 may include a first removal section 122i and a second removal section 122o. As shown in the depicted embodiment, the first section122i is disposed nearest to the electrode patch and the second section 122o is disposed around the first section 122i. While Figure 2 illustrates two sections 122i, 122o for removal, it should be appreciated that the adhesive system 120 may include one section 122 for removal or a plurality of sections, such as three, four, five tear-away sections.

In an illustrative example, the patient places the wearable monitor 100 onto their skin. Overtime, the outer edge of the second section 122o gradually lifts and fails. To prevent this failure from propagating and leading to other areas of the adhesive system 120, the patient removes the second section 122o, providing a new edge of the first section 122i that is firmly adhered to the patient's skin without any failure. This process may be repeated with the first section 122i. Namely, the outer edge of the first section 122i may gradually lift and fail. To prevent this failure from propagating, the patient may remove the first section 122i, providing a new edge of the electrode patch 104 that is firmly adhered to the patient's skin without any failure. This illustrative example demonstrates how the adhesive system 120 may increase the monitoring period, collecting additional data, and improving diagnostic efficacy.

For ease of removal, each of the plurality of sections 122 may include a tear-away tab 128. For example, the patient may grip a tear-away tab 128 to remove a respective section 122. The width of the tear-away tab 128 may be greater than a width of the remainder of the section 122. In further embodiments, the location, size, and shape of the tear-away tabs 128 may be adjusted. In some examples, the section 122 is coupled with the electrode patch 104 at the tear-away tab 128.

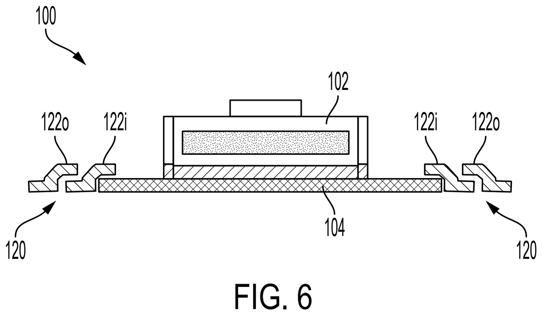

As shown in Figure 2, the one or more sections 122 of the adhesive system 120 may be partially or fully disposed around a perimeter of the electrode patch 104. This can provide multiple fresh edges of the wearable monitor 100. In some embodiments, the adhesive system 120 (e.g., the one or more sections of the adhesive system 120) interface with at least 50%, 60%, 70%, or 80% of the perimeter of the electrode patch 104. The sections 122 may have the same outer shape as the electrode patch 104 or may have a different shape. Each section 122 may interface with the electrode patch 104 as shown in Figure 2, so that either one of the sections 122 may be removed while the other sections 122 are still connected to the electrode patch. As will be discussed in greater detail in relation to Figures 6-9, the sections 122 for removal may be coplanar with the electrode patch 104 or may overlap with the electrode patch 104.

The adhesive system 120 and the electrode patch 104 may be composed of the same material or a different material. For example, the adhesive system 120 and/or the electrode patch 104 may be constructed of a wearable gauze, latex, or similar wrap knit or stretchable and wear-safe material, such as a Tricot-type linen with a pressure sensitive adhesive (PSA) on the inner side, or contact surface. The adhesive may be a non-irritating adhesive, such as hydrocolloid, to facilitate long-term wear. The hydrocolloid, for instance, is typically made of mineral oil, cellulose and water and lacks any chemical solvents, so should cause little itching or irritation.

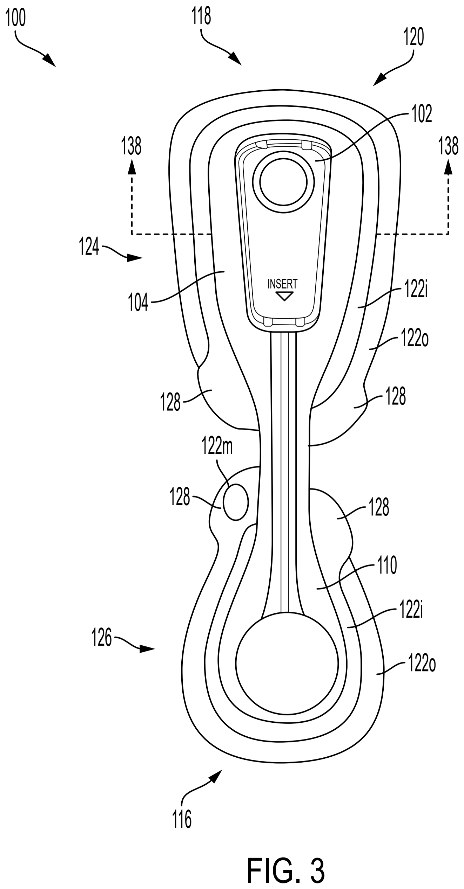

Figure 3 illustrates an adhesive system 120 according to an example of the present disclosure. As discussed previously, the reliability of the wearable monitor 100 may be dependent on the contact of the one or more electrodes with the skin. In some embodiments, the wearable monitor 100 may include an adhesive system 120 for each electrode as shown in the depicted embodiment. Accordingly, if the adhesive of one electrode begins to fail, the failure will not propagate to the other electrode. This may ensure that the electrode contact is maintained.

For example, as shown in Figure 3, the adhesive system may include a distal portion 124 and a proximal portion 126. The distal portion 124 of the adhesive system 120 is disposed around the distal end 118 of the electrode patch 104, which may include a first electrode. Additionally, the distal end 118 of the electrode patch 104 includes the monitor recorder 102. The proximal portion 126 of the adhesive system 120 is disposed around the proximal end 116 of the electrode patch 104, which may include a second electrode. In an example embodiment, the adhesive system 120 includes a plurality of sections 122 configured to be removed or torn away. Particularly, each of the distal portion 124 and the proximal portion 126 of the adhesive system 120 includes a first section 122i and a second section 122o for removal. Additionally, each of the sections 122i, 122o may include a tear-away tab 128 with a greater width than the remainder of the section 122i, 122o. In an outward direction, the wearable monitor 100 includes the electrode patch 104, the first section 122i, and the second section 122o.

As shown in Figure 3, the sections 122 for removal on the proximal portion 126 of the adhesive system 120 function independently of the sections 122 for removal on the distal portion 124 of the adhesive system 120. For example, the second section 122o of the distal portion 124 may be removed without removing the second section 122o of the proximal portion 126. Likewise, the first section 122i of the distal portion 124 may be removed without removing the first section 122i of the proximal portion 126. This may be advantageous when a failure of the adhesive system 120 is concentrated at one end of the electrode patch 104. In certain embodiments, electrode patch 104 is oriented vertically, along the sternum; thus, in certain embodiments, adhesive failure may be limited to one of the two ends and the independence of sections 122 is desirable.

For example, the second section 122o of the distal portion 124 may fail while the second section 122o of the proximal portion 126 remains firmly adhered to the patient. Thus, rather than the patient having to remove the second section 122o of the proximal portion 126 (that remains firmly adhered without any failure), the patient only needs to remove the section 122 where the failure is located (i.e., the second section 122o of the distal portion 124).

In another embodiment, the number or shape of the sections 122 for removal may be varied to address common failures. For example, if the adhesive system 120 is more likely to fail at the proximal portion 126, the proximal portion 126 may include one or more sections 122 in addition to the described first section 122i and second section 122o. Common failures may also occur where anatomical features of the patient create an uneven surface, which can create a gap between the adhesive and the skin. In some embodiments, the anatomical feature may include a mole, a wound, or some other protrusion on the surface of the skin, such as a protruding xiphoid process along the bottom of the sternum. Removal of a section 122m of material of the adhesive system 120 can prevent failures due to the uneven surface. For example, section 122m may be removed so that a mole or other skin feature can "pop through" the adhesive system 120 without undesirably tenting or distorting the adhesive system 120.

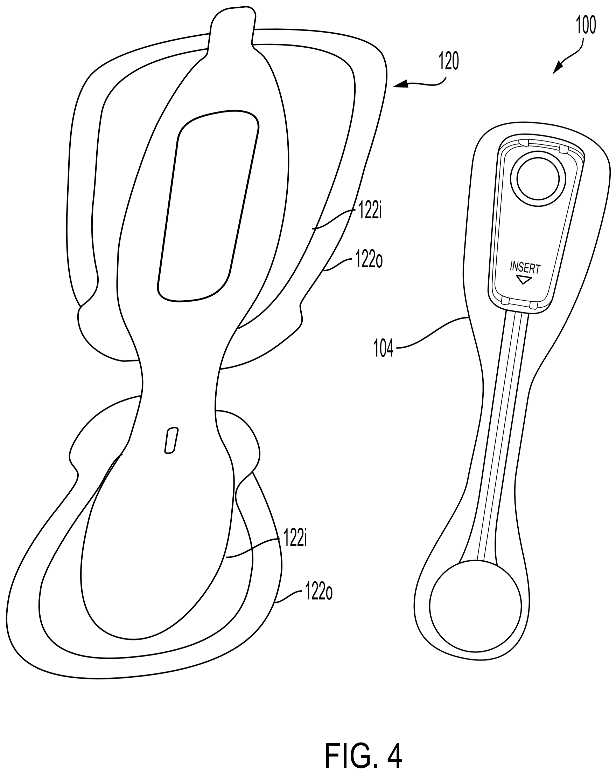

In some embodiments, the adhesive system 120 is an extension of the electrode patch 104, as shown in Figures 2 and 3. In other embodiments, as depicted in Figure 4, the adhesive system 120 may be applied in addition to the electrode patch 104. For example, the electrode patch 104 may be applied to the patient's skin using the adhesive on the inner side of the electrode patch 104. The adhesive system 120 may be applied over the wearable monitor 100 including the electrode patch 104 for additional support. The adhesive system 120 may be applied immediately after application of the electrode patch 104. Alternatively, the adhesive system 120 may be applied after an edge of the electrode patch 104 begins to propagate to extend the life of the wearable monitor 100. In this embodiment, adhesive system 120 includes apertures or cutouts for various structure associated with electrode patch 104; adhesive system 120 is applied "on top of" the electrode patch 104, such that it overlaps the edge of electrode patch 104 and provides the related benefits previously described herein.

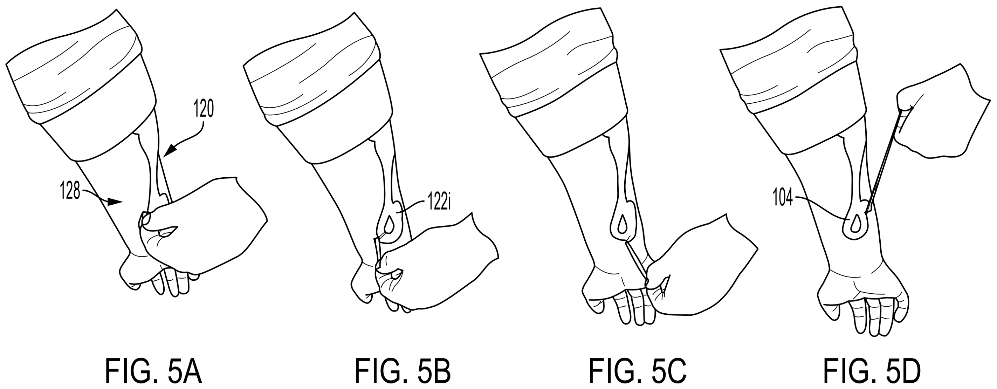

Figures 5A to 5D illustrate an example process of a section being removed from an adhesive system, according to various embodiments. As illustrated, the patient grips the section, such as at the tear-away tab 128 of the section. With sufficient force, the adhesive on the inner side of the adhesive system 120 may be overcome, thereby removing the first section 122i from the adhesive system 120. As the first section 122i is removed, the edge of the electrode patch 104 is revealed, firmly adhered to the patient's skin.

Figure 6 illustrates an example embodiment of a cross-sectional view of the extended wear electrocardiography monitor of Figure 3 taken at cross-section 138. Figure 6 illustrates the wearable monitor 100 having a monitor recorder 102 disposed on the electrode patch 104 with an adhesive system 120. As described in reference to Figure 2 to Figure 5D, the adhesive system 120 includes a first section 122i and a second section 122o for removal. While Figure 6 illustrates the distal end of the electrode patch 104, it should be appreciated that the foregoing description likewise may be applied to the proximal portion 126 of the adhesive system 120.

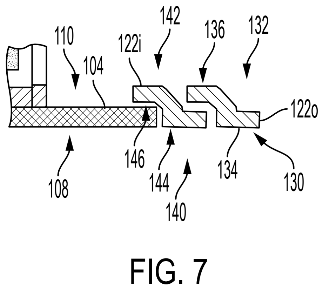

Figure 7 illustrates a detailed view of the adhesive system of the extended wear electrocardiography monitor of Figure 5, according to various embodiments. As shown in the depicted embodiment, the first section 122i overlaps the electrode patch 104 and the second section 122o overlaps the first section 122i. The second section 122o includes an inner surface 130 and an outer surface 132. The inner surface 130 faces the skin of the patient, corresponding to the inner side 108 of the electrode patch 104. The outer surface 132 faces outward from the patient, away from the skin, corresponding to the outer side 110 of the electrode patch 104. The inner surface 130 of the second section 122o includes a contact portion 134 and an overlap portion 136. The contact portion 134 may include an adhesive surface that adheres to the patient's skin. As previously described, the edge of the second section 122o may gradually lift, leading to failure of the adhesive system 120. To prevent this failure from propagating and leading to other areas of the adhesive system 120, the patient removes the second section 122o, providing a new edge of the first section 122i that is firmly adhered to the patient's skin without any failure. To provide ease of removal, the overlap portion 136 may include an adhesive surface that differs from the adhesive surface of the contact portion 134. It should be appreciated that, in other embodiments, overlap portion 136 and contact portion 134 have the same type of adhesive.

In an example embodiment, the overlap portion 136 and the contact portion 134 may include an adhesive with a different removal force. The overlap portion 136 may include a waxy adhesive that adheres less firmly to the first section 122i than the adhesive surface of the contact portion 134 adheres to the skin of the patient. Stated differently, the contact portion 134 may have a stronger adhesive than the overlap portion 136. This configuration may be advantageous when removing the second section 122o without disturbing the first section 122i. Namely, the contact portion 134 provides a strong adherence to the patient's skin while the overlap portion 136 adheres to the first section 122i less firmly. Thus, removal of the second section 122o is less likely to cause the first section 122i to be unintentionally removed from the adhesive system 120 when the patient removes the second section 122o from the adhesive system 120.

The first section 122i has a similar structure that functions in an identical manner as the second section 122o. The first section 122i includes an inner surface 140 and an outer surface 142. The inner surface 140 faces the skin of the patient, corresponding to the inner side 108 of the electrode patch 104. The outer surface 142 faces outward from the patient, away from the skin, corresponding to the outer side 110 of the electrode patch 104. The inner surface 140 of the first section 122i includes a contact portion 144 and an overlap portion 146. The contact portion 134 may include an adhesive surface that adheres to the patient's skin. As previously described, the edge of first section 122i may gradually lift, leading to failure of the adhesive system 120. To prevent this failure from propagating and leading to other areas of the adhesive system 120, the patient removes the first section 122i, providing a new edge of the electrode patch 104 that is firmly adhered to the patient's skin without any failure. To provide ease of removal, the overlap portion 146 may include an adhesive surface that differs from the adhesive surface of the contact portion 144.

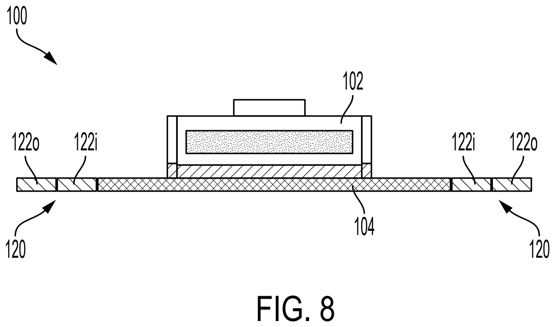

Figure 8 illustrates another example embodiment of a cross-sectional view of the extended wear electrocardiography monitor of Figure 3 taken at cross-section 138. Figure 8 illustrates the wearable monitor 100 having a monitor recorder 102 disposed on the electrode patch 104 with an adhesive system 120. As described in reference to Figure 2 to Figure 5D, the adhesive system 120 includes the first section 122i and the second section 122o. It should be appreciated that the foregoing description likewise applies to the proximal portion 126 of the adhesive system 120.

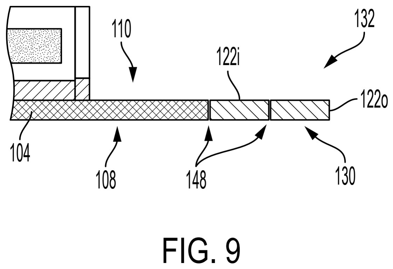

Figure 9 illustrates a detailed view of an adhesive system of the extended wear electrocardiography monitor of Figure 8, according to various embodiments. The second section 122o includes an inner surface 130 and an outer surface 132. The inner surface 130 faces the skin of the patient, corresponding to the inner side 108 of the electrode patch 104. The outer surface 132 faces outward from the patient, away from the skin, corresponding to the outer side 110 of the electrode patch 104. The edge of second section 122o may gradually lift, leading to failure of the adhesive system 120. To prevent this failure from propagating and leading to other areas of the adhesive system 120, the patient may remove the second section 122o, providing a new edge of the first section 122i that is firmly adhered to the patient's skin without any failure.

In the depicted embodiment, the first and second sections 122i, 122o are coplanar with each other and with the electrode patch. To provide ease of removal, the interface of the second section 122o and the first section 122i may include a plurality of perforations 148 at the boundary or interface between first section 122i and second section 122o. The plurality of perforations 148 allow the second section 122o to be easily removed from the first section 122i without unintentionally removing the first section 122i. Further, the interface of the first section 122i and the electrode patch 104 may include the plurality of perforations 148. Similarly, the plurality of perforations 148 allow the first section 122i to be easily removed from the electrode patch 104 without unintentionally removing the electrode patch 104.

The plurality of perforations 148 of the adhesive system 120 may be in a predefined shape. For example, as discussed previously, the plurality of perforations 148 may be machined onto the adhesive system 120 in locations known to cause common failures, such as uneven surfaces of the skin. In some embodiments, the plurality of perforations 148 may allow a customizable adhesive system.

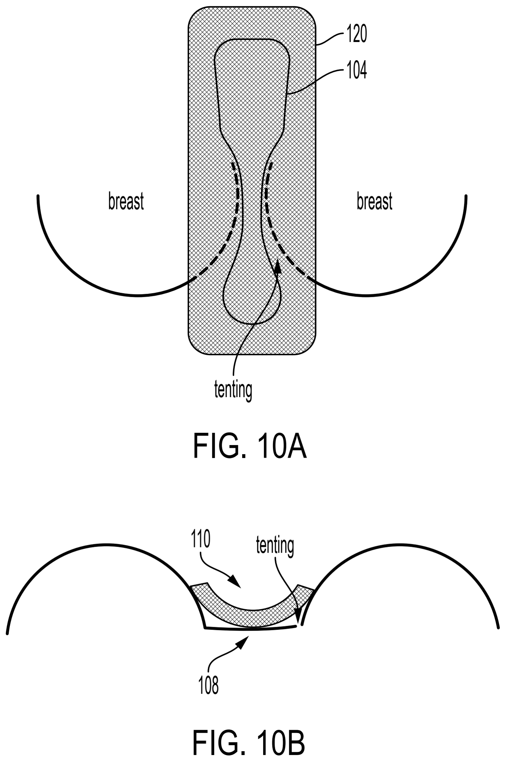

Figures 10A to 10B illustrates an extended wear electrocardiography monitor having a customizable adhesive system, according to various embodiments. As previously introduced, the electrode patch 104 includes an inner side 108 and an outer side 110. When coupled to the patient, the inner side 108 contacts and adheres to the patient's skin while the outer side 110 faces outward, away from the from the patient's skin. Without proper attachment of the electrode patch 104 to the patient, the electrode patch 104 cannot collect physiological signals and the monitor recorder will not receive physiological signals for processing, transmission, or diagnostic procedures. In turn, the patient cannot benefit from the application of the wearable monitor 100.

Improper attachment may arise when the adhesive system is inappropriately shaped or sized for the patient, leading to various issues. For example, the adhesive system may irritate the skin due to contact or coverage with features of the skin, such as a mole or a wound. Further, nonessential material of the adhesive system may increase the susceptibility of folding or wrinkling that may reduce the integrity of the adhesive system. Additionally, excessive material may cause the adhesive system to interfere with other devices or the patient's clothes. The patient may be forced to apply the adhesive system to discontinuous skin surface. For example, the discontinuous surface may include the skin of the sternum and the skin of the side of breast (as illustrated in Figure 10A) or an anatomical landmark, such as a mole, a wound, or a protruding xiphoid. Such discontinuous application may increase susceptibility to ingress of moisture, liquids, or particles. Discontinuous application of the adhesive system may also make the adhesive system more susceptible to shear or peel forces caused by the relative motion between the different, discontinuous surfaces.

Figure 10B illustrates an extended wear electrocardiography monitor having a customizable adhesive system with tenting, according to various embodiments. Tenting is one obstacle that prevents the electrode patch 104 from properly attaching to a patient's skin. Tenting, which may be caused by nonessential material of the adhesive system, may lead to any number of negative effects, as detailed above. In reference to Figure 10B, the inner side 108 of the adhesive system 120 contacts discontinuous surfaces of the patient. Namely, the adhesive system 120 is coupled to the patient's sternum and sides of each breast, leading to tenting of the adhesive system 120; having a narrower midsection of the adhesive system 120 would be desirable, so as to avoid tenting. It should be appreciated that Figure 10B provides one representative example of discontinuous attachment, and such improper attachment may occur in many different situations, negatively affecting the attachment of the adhesive system 120 to the patient.

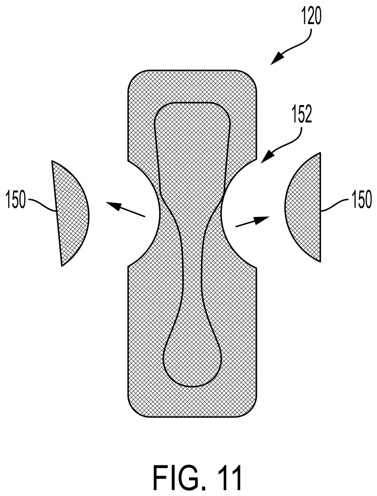

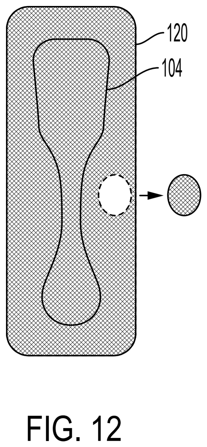

Figures 11 and 12 illustrate an extended wear electrocardiography monitor having nonessential material removed from a customizable adhesive system, according to various embodiments. To prevent tenting and provide proper attachment between the adhesive system 120 and the patient's skin, the adhesive system 120 may be customizable. For example, Figure 11 illustrates the removal of nonessential material 150 that may cause tenting, as shown previously in Figure 10B. Further, Figure 12 illustrates the removal of nonessential material 150 that would otherwise cover a portion of the patient's skin that is known to interfere with the adhesive, such as mole. To facilitate the removal of nonessential material, the adhesive system 120 may include a plurality of perforations 152 for customizable detachment of nonessential material 150. The plurality of perforations 152 may have a variety of sizes, shapes, locations, and patterns. The plurality of perforations 152 may be manufactured through either additive or subtractive fabrication.

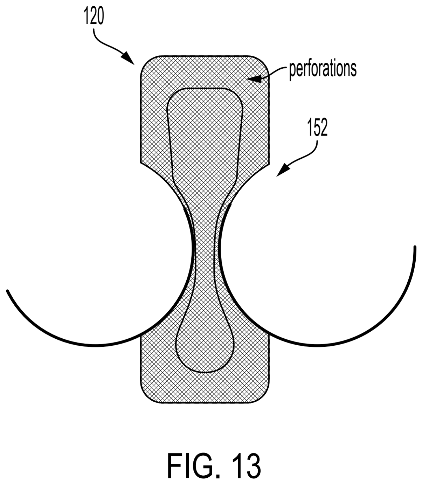

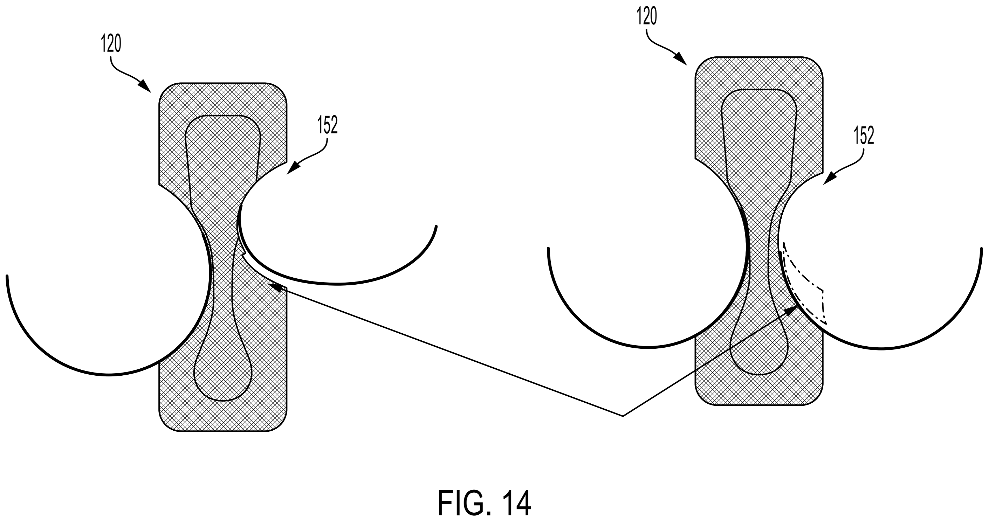

Figure 13 illustrates an extended wear electrocardiography monitor having a customizable adhesive system as applied to a patient's skin, according to various embodiments. Figure 13 further illustrates the result of removing nonessential material from the adhesive system 120. With nonessential material removed, the adhesive system 120 attaches to the patient's skin with a continuous application. As shown in Figure 13, the adhesive system 120 does not have tenting, unlike the application of the adhesive system 120 in Figure 10B. Figure 13 illustrates an extended wear electrocardiography monitor having a customizable adhesive system as applied to a patient's skin, according to various embodiments. Figure 14 illustrates another result of removing nonessential material from the adhesive system 120. With the fully customizable removal of nonessential material, the adhesive system 120 can be applied to asymmetrical surfaces, such as asymmetrical breasts. As shown in Figure 14, nonessential material is removed to match the geometry of the skin surfaces. Similar to the application of the adhesive system 120 of Figure 13, the adhesive system 120 of Figure 14 does not have tenting with the removal of nonessential material.

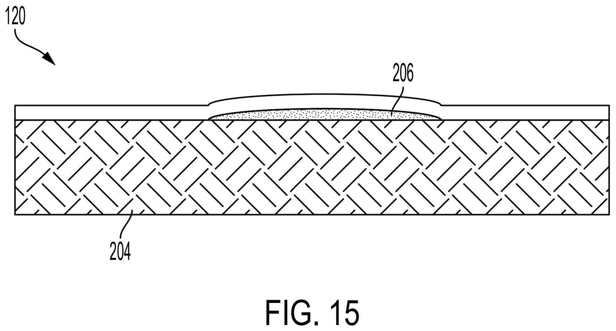

In addition or alternatively to aiding in the removal of nonessential material, the perforations of the adhesive system create an unimpeded conduit, providing an egress for moisture, perspiration, or skin oils, thereby reducing a buildup between the adhesive system and the skin. Additionally, one or more openings, such as removal of section 122m discussed in Figure 3 above, in the adhesive system (in addition to or alternatively to the perforations) could also provide an egress for moisture. Figure 15 illustrates a side view of an adhesive system without perforations or openings that is coupled to skin during perspiration. The adhesive system 120 fails to provide an egress for perspiration 206. Instead, perspiration 206 pools between the adhesive system 120 and skin 204. Namely, the pooling of moisture from perspiration causes the adhesive surface to separate from the skin. As a result, the adhesive system 120 may prematurely fail due to bond degradation or failed patient compliance due to discomfort.

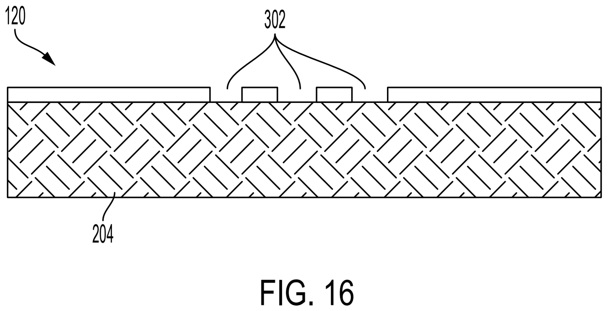

Figure 16 illustrates, by way of example, a side view of an adhesive system 120, according to an example embodiment of the present disclosure. The adhesive system 120 includes a plurality of openings 302. The plurality of openings 302 may provide increased breathability between a medical device and patient skin. Additionally, the plurality of openings 302 may interrupt or reduce the transmission of tensile and peel forces across the adhesive system 300, preventing failure from patient movement.

As shown in Figure 16, the plurality of openings 302 includes three openings that are circular in shape. However, the number, shape, size, density, arrangement, and location of the plurality of openings 302 can be altered to achieve desired performance characteristics. Such performance characteristics may relate to preferential or biased deformation directions, the amount of deformation under stress, or breathability of the adhesive system. For example, the characteristics of the plurality of openings 302 may provide elasticity in one direction while preventing deformation in another direction.

In various embodiments, the plurality of openings 302 may comprise the following geometrical shapes: ellipse, triangle, rectangle, hexagonal, tear drop, star, scallop, zig-zag, chevron, zipper, spider web (concentric shapes connected together by one or more connections). The plurality of openings 302 may also comprise non-geometric shapes. In an example, various tools can be used to construct a plurality of openings 302 without a regular size or shape. The plurality of openings 302 may also correspond to shapes that represent an open cell foam or a sponge-like structure. In another example, the plurality of openings 302 may comprise a slit shape, which include straight, splined, cross/pie, branching, scallop tear drop, wavy, or zig zag. Any number of patterns and shapes may be combined in any combination of densities.

The plurality of openings' sizes, shapes, locations and densities may be used to optimize where and to what extent the adhesive system elastically or plastically deforms within a given region of the system. A plurality of openings sizes, shapes, locations, and densities may also be used to optimize the ratio of adhesive bond surface area to edge length (total length of all internal and external profile edges of the adhesive).

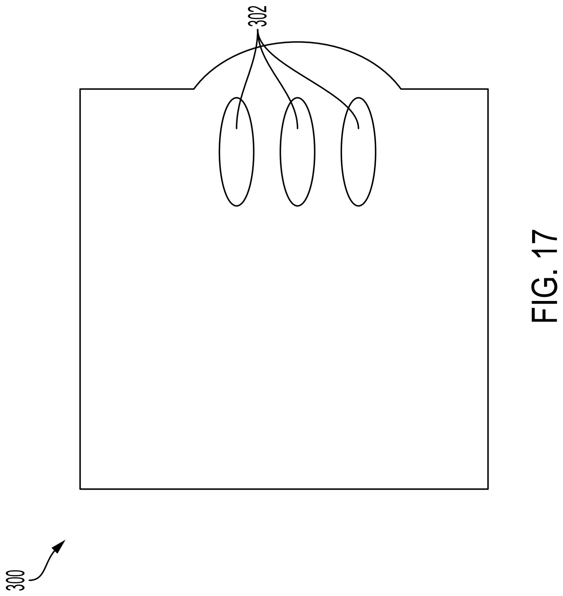

Figure 17 illustrates, by way of example, a side view of an adhesive system under localized stress, according to an example embodiment of the present disclosure. Under stress, the plurality or openings 302 deform from their original circular shape. Specifically, the adhesive system 120 under localized stress concentrates the stress to the location of the plurality of openings 302. This stress localization may be advantageous by providing localized control of the bulk materials properties.

In many embodiments, the thickness of the adhesive system 120 may be varied. For example, the thickness of the adhesive system 120 may be between 1 and 50 millimeters. When, for example, the adhesive system 120 comprises a laminate of layers, the geometry or pattern of each layer can be varied to affect desired properties. The diameter of each of the plurality of openings 302 may also be varied. In an example, the diameter may be several microns while still providing an egress for moisture and patient perspiration. In another example, the diameter may be determined based on the minimum spacing required to prevent the openings adhesive system 400 from breaking or tearing under stress.

Further features of the disclosure are defined in the following list of numbered clauses, and numbered sub-clauses.

Clause 1: An adhesive system for a wearable medical device comprising: an electrode patch comprising one or more electrodes for sensing a signal indicative of a physiological characteristic of a patient; a removable section disposed at least partially around a perimeter of the electrode patch, wherein the removable section comprises an adhesive; and wherein the removable section is configured to be removed from the adhesive system.

Clause 2: The adhesive system of clause 1, wherein the removable section comprises a first removable section and a second removable section, wherein the first removable section is disposed at least partially around a perimeter of the electrode patch and the second removable section is disposed at least partially around a perimeter of the first removable section.

Clause 3: The adhesive system of clause 1 or 2, wherein the electrode patch comprises a distal end and a proximal end, wherein the removable section includes: a distal removable section disposed around the distal end of the electrode patch; and a proximal removable section disposed around the proximal end of the electrode patch.

Clause 4: The adhesive system of clause 3, wherein the distal end of the electrode patch comprises a first electrode and the proximal end of the electrode patch comprises a second electrode.

Clause 5: The adhesive system of clause 3 or 4, wherein the distal removable section and the proximal removable section are independently removable.

Clause 6: The adhesive system according to any preceding clause, wherein the removable section comprises: a contact portion having a contact inner surface, wherein the contact inner surface is configured to adhere to skin of a patient; and an overlap portion having an overlap inner surface, wherein the overlap inner surface is configured to adhere to the electrode patch.

Clause 7: The adhesive system according to clause 6, wherein the contact inner surface has a stronger adhesive than the overlap inner surface, such that the overlap inner surface is permanently adhered to the electrode patch.

Clause 8: The adhesive system according to any preceding clause, wherein the removable section comprises one or more tabs, wherein the removable section has a thickest width at the one or more tabs.

Clause 9: The adhesive system according to clause 8, wherein the one or more tabs comprises a first tab and a second tab.

Clause 10: The adhesive system according to any preceding clause, wherein the electrode patch and the removable section are co-planar.

Clause 11: The adhesive system according to clause 10, wherein a plurality of perforations are disposed on an interface of the electrode patch and the removable section.

Clause 12: The adhesive system according to any preceding clause, wherein the removable section overlaps a portion of the perimeter of the electrode patch.

Clause 13: The adhesive system according to any preceding clause, further comprising an additional removable section surrounded by a plurality of perforations, wherein the additional removable section creates an opening when removed from the adhesive system.

Clause 14: A wearable medical device comprising: an electrode patch comprising one or more electrodes for sensing a signal indicative of a physiological characteristic of a patient; a monitor recorder having a microcontroller, the monitor recorder configured to receive the signal from the one or more electrodes; and an adhesive system comprising an adhesive configured to adhere to a skin of the patient, wherein the adhesive system is disposed at least partially around a perimeter of the electrode patch, and wherein the adhesive system comprises a section that is configured to be removed from the adhesive system.

Clause 15: The wearable medical device according to clause 14, wherein the monitor recorder is removably coupled to the electrode patch.

Clause 16: The wearable medical device according to clause 14 or 15, wherein the section configured to be removed comprises a first removable section and a second removable section, wherein the first removable section is disposed at least partially around a perimeter of the electrode patch and the second removable section is disposed at least partially around a perimeter of the first removable section.

Clause 17: The wearable medical device according to clause 16, wherein the adhesive system comprises a plurality of perforations at an interface of the first removable section and the second removable section.

Clause 18: The wearable medical device according to clause 16 or 17, wherein the first removable section and the second removable section are co-planar.

Clause 19: The wearable medical device according to any of clauses 14 to 18, wherein the adhesive system overlaps the electrode patch.

Clause 20: The wearable medical device according to any of clauses 14 to 19, wherein the adhesive system overlaps an entire perimeter of the electrode patch.

While the invention has been particularly shown and described as referenced to the embodiments thereof, those skilled in the art will understand that the foregoing and other changes in form and detail may be made therein without departing from the spirit and scope.

## Claims

1. An adhesive system for a wearable medical device comprising:
an electrode patch comprising one or more electrodes for sensing a signal indicative of a physiological characteristic of a patient;
a removable section disposed at least partially around a perimeter of the electrode patch, wherein the removable section comprises an adhesive; and
wherein the removable section is configured to be removed from the adhesive system.

2. The adhesive system of claim 1, wherein the removable section comprises a first removable section and a second removable section, wherein the first removable section is disposed at least partially around a perimeter of the electrode patch and the second removable section is disposed at least partially around a perimeter of the first removable section.

3. The adhesive system of claim 1 or 2, wherein the electrode patch comprises a distal end and a proximal end,
wherein the removable section includes:
a distal removable section disposed around the distal end of the electrode patch; and
a proximal removable section disposed around the proximal end of the electrode patch.

4. The adhesive system of claim 3, wherein the distal end of the electrode patch comprises a first electrode and the proximal end of the electrode patch comprises a second electrode.

5. The adhesive system of claim 3 or 4, wherein the distal removable section and the proximal removable section are independently removable.

6. The adhesive system of any preceding claim, wherein the removable section comprises:
a contact portion having a contact inner surface, wherein the contact inner surface is configured to adhere to skin of a patient; and
an overlap portion having an overlap inner surface, wherein the overlap inner surface is configured to adhere to the electrode patch, optionally, wherein the contact inner surface has a stronger adhesive than the overlap inner surface, such that the overlap inner surface is permanently adhered to the electrode patch.

7. The adhesive system according to any preceding claim, wherein the removable section comprises one or more tabs, wherein the removable section has a thickest width at the one or more tabs, optionally, wherein the one or more tabs comprises a first tab and a second tab.

8. The adhesive system according to any preceding claim, wherein the electrode patch and the removable section are co-planar, optionally, wherein a plurality of perforations are disposed on an interface of the electrode patch and the removable section.

9. The adhesive system according to any preceding claim, wherein the removable section overlaps a portion of the perimeter of the electrode patch.

10. The adhesive system according to any preceding claim, further comprising an additional removable section surrounded by a plurality of perforations, wherein the additional removable section creates an opening when removed from the adhesive system.

11. A wearable medical device comprising:
an electrode patch comprising one or more electrodes for sensing a signal indicative of a physiological characteristic of a patient;
a monitor recorder having a microcontroller, the monitor recorder configured to receive the signal from the one or more electrodes; and
an adhesive system comprising an adhesive configured to adhere to a skin of the patient, wherein the adhesive system is disposed at least partially around a perimeter of the electrode patch, and wherein the adhesive system comprises a section that is configured to be removed from the adhesive system.

12. The wearable medical device according to claim 11, wherein the monitor recorder is removably coupled to the electrode patch.

13. The wearable medical device according to claim 11 or 12, wherein the section configured to be removed comprises a first removable section and a second removable section, wherein the first removable section is disposed at least partially around a perimeter of the electrode patch and the second removable section is disposed at least partially around a perimeter of the first removable section.

14. The wearable medical device according to claim 13, wherein the adhesive system comprises a plurality of perforations at an interface of the first removable section and the second removable section, optionally wherein the first removable section and the second removable section are co-planar.

15. The wearable medical device according to any one of claims 11 to 14, wherein the adhesive system overlaps the electrode patch, optionally wherein the adhesive system overlaps an entire perimeter of the electrode patch.
